# EUROPEAN PATENT APPLICATION

(11) **EP 1 506 772 A2**
(43) Date of publication of application: **16.02.2005**
(21) Application number: 04291385.5
(22) Date of filing: 03.06.2004
(51) Int. Cl.: A61K 7/42

(54) **Aesthetically and SPF improved UV sunscreens comprising glass microspheres**

(30) Priority: 13.08.2003 US 639586
(71) Applicant: L'OREAL, 75008 Paris (FR)
(72) Inventor: Hansenne, Isabelle, 07090 New Jersey (US); Rick, Donald, Dumont NJ 07628 (US)
(74) Representative: Miszputen, Laurent

(57) **Abstract**

The present invention relates to a topically applicable, aesthetically pleasing/elegant cosmetic/dermatological sunscreen compositions well suited for both effective and SPF-improved UV-photoprotection of human skin, scalp, lips, mucous membranes and/or hair contain (a) at least one UV-A sunscreen and/or at least one UV-B sunscreen and (b) at least glass microspheres, formulated into (c) a topically applicable, cosmetically/dermatologically acceptable vehicle, diluent or carrier therefor.

The present invention relates also to glass microspheres in a topically applicable cosmetic/dermatological sunscreen product for UV-photoprotecting human skin, scalp, lips, mucous membranes and/or hair against the damaging effects of ultraviolet irradiation for improving the aesthetics and SPF values thereof.

## Description

The present invention relates to novel cosmetic/dermatological compositions for topical application for the ultraviolet (UV)-photoprotection of the skin, scalp, lips, mucous membranes and/or hair against the damaging effects of UV radiation, in particular solar radiation.

This invention more especially relates to novel aesthetically elegant and SPF-improved sunscreen formulations containing glass, notably borosilicate beads that have a soft, nongreasy and nontacky feel when applied onto the skin and when compared to identical formulations not containing the glass/borosilicate beads.

It is well known to this art that light radiation of wavelengths of from 280 nm to 400 nm promotes tanning of the human epidermis, and that irradiation of wavelengths of from 280 nm to 320 nm, i.e., UV-B irradiation, causes erythema and burning of the skin which can impair the development of a natural tan; hence, such UV-B radiation must thus be screened from the skin.

It is also known to this art that UV-A radiation, of wavelengths from 320 to 400 nm, which tans the skin, also adversely affects it, in particular in the case of sensitive skin or a skin which is continually exposed to solar radiation.

UV-A rays cause, in particular, a loss in the elasticity of the skin and the appearance of wrinkles, promoting a premature aging thereof. Such irradiation promotes triggering of the erythematous reaction or enhances this reaction in certain individuals, and may even be the source of phototoxic or photoallergic reactions. Thus, it is desirable to also screen our UV-A radiation.

A wide variety of cosmetic compositions intended for the photoprotection (UV-A and/or UV-B) of human skin is known to this art.

These photoprotective/sunscreen compositions are quite often oil-in-water emulsions (namely, a cosmetically acceptable vehicle, carrier or diluent comprising an aqueous continuous dispersing phase and an oily discontinuous dispersed phase) which contains, in various concentrations, one or more standard lipophilic and/or hydrophilic organic sunscreen compounds capable of selectively absorbing harmful or deleterious UV radiation. These sunscreen compounds (and the amounts thereof) are selected as a function of the desired sun protection factor (the sun protection factor (SPF) being expressed mathematically by the ratio of the irradiation time required to attain the erythema-forming threshold with the UV screening agent to the time required to attain the erythema-forming threshold in the absence of UV screening agent).

Too, there exists an increasing demand for higher SPF suncare products, but which also have elegant product aesthetics. However, this is difficult, as high SPF sunscreen compositions contain high levels of sunscreen actives that result in the formulations having negative or objectionable product aesthetics, such as greasiness, tackiness and stickiness.

Also, as hereinafter will be seen, the present invention features formulating glass microspheres into sunscreen compositions for improving the aesthetics and SPF values thereof. WO 03/045345 Al describes certain cosmetic products containing inorganic fillers and active ingredients, but the optically clear glass particles comprised thereof are necessarily ground and heterogeneous versus the regularly homogeneous microspheres uniformly distributed throughout the formulations of the invention.

And Japanese Application No. 05-147115, filed May 14, 1993 and published as Kokai No. 06-321726 on November 22, 1994, relates to certain makeup compositions combining hollow or solid glass beads with metal oxides (titanium, zinc, iron) characteristically to enhance the mechanical properties thereof. These prior art necessarily makeup compositions include face powders and foundations, as well as pre-shave makeups, but do not comprehend the aesthetically and SPF improved UV-sunscreens provided consistent herewith.

Thus, it has now surprisingly and unexpectedly been determined that by formulating glass microspheres into sunscreen compositions, the final product formulations are aesthetically elegant and have a soft, nongreasy and nontacky feel when topically applied onto skin, scalp, lips, mucous membranes and/or hair and when compared to identical formulations not containing the glass microspheres.

It has also now unexpectedly and surprisingly been determined that the addition of borosilicate/glass beads increased the *in vitro* SPF of the sunscreen compositions.

The Figure of Drawing is a graph reflecting the average *in vitro* SPFs of seven (7) different sunscreen formulations, four (4) not according to the present invention and three (3) in accordance herewith.

More particularly according to the present invention, aesthetically pleasing, dry, nongreasy and nontacky sunscreen formulations exhibiting enhanced SPF are provided by incorporating therein a thus effective amount of glass microspheres, notably calcium aluminum borosilicate or sodium borosilicate beads.

The above phenomena were unexpected, as previously the aesthetics of a sunscreen product were improved via formulating therein methyl methacrylate crosspolymer beads, but at the expense of reducing the efficacy of the sunscreen formulation. Indeed, a reduction of *in vivo* SPF by as much as 30% has been observed with the addition of Ganzpearl GMP-0820.

Ganzpearl GMP-0820 is marketed by Presperse Inc. and essentially consists of beads of average size 10 µm which improves the aesthetics of sunscreen formulations by significantly reducing the tackiness and greasiness thereof.

However, incorporation of the Ganzpearl GM-0820 into sunscreen formulations did not provide the advantages of aesthetic elegance and enhanced SPF according to the present invention.

The glass microspheres according to the invention are essentially homogeneous and essentially uniform in sphericity. The mean particle size of the subject microbeads can range up to 60-70 µm, but typically is on the order of 10-20 µm.

Preferred cosmetic glass microspheres according to this invention include those amorphous hollow microspheres of calcium aluminum borosilicate marketed by Presperse Inc. under the trademark LUXSIL®. It is a high slip, chemically inert, non-absorbent, non-porous free-flowing white powder, essentially uniformly spherical in form, having a mean particle size ranging from 9-13 microns. The typical particle size distribution thereof is as follows:

| | |
|---|---|
| 10% | 3-6 µm |
| 50% | 8-12 µm |
| 90% | 16-23 µm |

Moreover, its density ranges from 1.05 - 1.15 g/cc, has a weight loss on drying of less than 0.5%, and contains less than 20 ppm of lead and less than 3 ppm of arsenic. Its CAS No. is 65997-17-3.

Preferred concentrations of the LUXSIL® free-flowing, spherical white powder microbeads is on the order of 0.1 % to 10% w/w in the formulation. Concentrations of from 1% to 5% are even more preferred.

Other suitable glass microspheres according to the present invention, whether hollow, solid or otherwise, include those sodium borosilicate particulates marketed by PQ Corporation under the trademark Q-CEL 570, having a granulometry on the order of 20 µm. Also suitable are those calcium/sodium borosilicate hollow microspheres marketed by 3M under the trademarks ES 22 and 1K, having a granulometry up to 70 µm, and 3M's Scotchlite K20 product, calcium/sodium borosilicate microspheres having a mean particle size on the order of 10 µm.

As aforesaid, other materials such as methyl methacrylate crosspolymer beads have improved the aesthetics of sunscreen formulations, but do not impart the advantageous characteristics of the present invention have reduced the efficacy of such products.

Presperse's Microsilk 418® product was also formulated, comprising beads of polyethylene + PTFE + synthetic was of average particle size of 10-12 µm. These did not improve the aesthetics of the formulation. Thus, not all spherical beads of average size on the order of 10 µm will improve product aesthetics.
Given the immediately aforesaid, it is even more unexpected that both aesthetics and efficacy are improved by formulating glass microspheres, such as those of calcium aluminum borosilicate or sodium borosilicate, into liquid UV-sunscreen compositions.

While not wishing to be bound to or by any particular theory or principle, it is believed that the subject glass microspheres enhance the efficacy of the sunscreen formulation by diffracting incoming light and increasing the likelihood of the incident light encountering a sunscreen molecule.

Vis-à-vis the ground glass particulates of the prior art, the glass microspheres of the present invention are essentially homogeneous in product morphology, are spheroidal in shape and are essentially homogeneously and uniformly distributed throughout the particular sunscreen formulation.

The cosmetic/dermatological compositions according to the present invention contain (a) at least one UV-A and/or UV-B sunscreen and (b) at least glass microspheres, formulated into (c) a topically applicable, cosmetically/dermatologically acceptable vehicle, diluent or carrier therefor.

By "UV-A and/or UV-B sunscreen" is intended any compound or any combination of compounds which, by mechanisms that are known per se of absorption and/or reflection and/or scattering of UV-A and/or UV-B radiation, prevents, or at least limits, the contact between such radiation and a surface (skin, hair) on which this or these compounds have been applied. Stated differently, these compounds may be UV-absorbing organic screening agents or inorganic (nano)pigments which scatter and/or reflect UV radiation, as well as mixtures thereof.

According to the present invention, the at least one UV-A and/or UV-B sunscreen may comprise one or more hydrophilic organic screening agents and/or one or more lipophilic organic screening agents and/or one or more mineral or inorganic (nano)pigments.

A preferred UV-photoprotecting agent according to the present invention is the dibenzoylmethane sunscreen avobenzone, or 4-(tert-butyl)-4 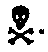 -methoxydibenzoylmethane, which is very well known to this art, is commercially available and is marketed, for example, under the trademark "PARSOL 1789" by 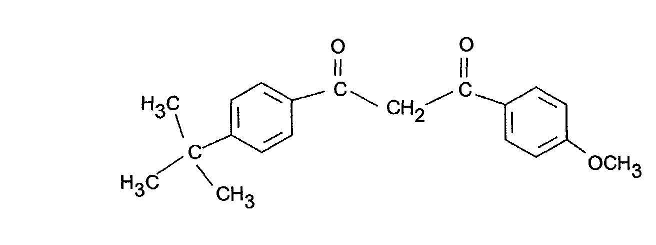 Givaudan. It has the structural formula:
Sunscreens according to the present invention which are physical blockers reflect or scatter ultraviolet radiation. Typical examples of physical blockers include red petrolatum, titanium dioxide, and zinc oxide. These physical blockers have been employed in a variety of suspensions and particle sizes and are frequently included in cosmetic formulations. A review of physical blockers may be found at "Sun Protection Effect of Nonorganic Materials," by S. Nakada & H. Konishi, **Fragrance Journal**, Volume 15, pages 64-70 (1987), which is incorporated by reference herein.

Sunscreens according to this invention which are chemical absorbers, like avobenzone, actually absorb harmful ultraviolet radiation. It is well known that chemical absorbers are classified, depending on the type of radiation they protect against, as either UV-A or UV-B absorbers. UV-A absorbers generally absorb radiation in the 320 to 400 nm region of the ultraviolet spectrum. UV-A absorbers include anthranilates, benzophenones, and dibenzoyl methanes. UV-B absorbers generally absorb radiation in the 280 to 320 nm region of the ultraviolet spectrum. UV-B absorbers include p-aminobenzoic acid derivatives, camphor derivatives, cinnamates, and salicylates.

Classifying the chemical absorbers generally as UV-A or UV-B absorbers is accepted within the industry. However, a more precise classification is one based upon the chemical properties of the sunscreens. There are eight major classifications of sunscreen chemical properties which are discussed at length in "Sunscreens - Development, Evaluation and Regulatory Aspects," by N. Shaath et al. 2nd. Edition, pages 269-273, Marcel Dekker, Inc. (1997). This discussion, in its entirety, is incorporated by reference herein.

The sunscreens which may be formulated according to the present invention typically comprise chemical absorbers, but may also comprise physical blockers. Exemplary sunscreens which may be formulated into the compositions of the present invention are chemical absorbers such as p-aminobenzoic acid derivatives, anthranilates, benzophenones, camphor derivatives, cinnamic derivatives, dibenzoyl methanes, β,β-diphenylacrylate derivatives, salicylic derivatives, triazine derivatives, benzimidazole compounds, bis-benzoazolyl derivatives, methylene bis-(hydroxyphenylbenzotriazole) compounds, the sunscreen polymers and silicones, or mixtures thereof. These are variously described in U.S. Patents Nos. 2,463,264, 4,367,390, 5,166,355 and 5,237,071 and in EP-0,863,145, EP-0,517,104, EP-0,570,838, EP-0,796,851, EP-0,775,698, EP-0,878,469, EP-0,933,376, EP-0,893,119, EP-0,669,323, GB-2,303,549, DE-1,972,184 and WO-93/04665, also expressly incorporated by reference. Also exemplary of the sunscreens which may be formulated into the compositions of this invention are physical blockers such as cerium oxides, chromium oxides, cobalt oxides, iron oxides, red petrolatum, silicone-treated titanium dioxide, titanium dioxide, zinc oxide, and/or zirconium oxide, or mixtures thereof.

A wide variety of sunscreens is described in U.S. Patent No. 5,087,445, issued to Haffey et al. on February 11, 1992; U.S. Patent No. 5,073,372, issued to Turner et al. on December 17, 1991; and Chapter VIII of **Cosmetics and Science and Technology** by Segarin et al. pages 189 et seq. (1957), all of which are incorporated herein by reference in their entirety.

Preferred among those sunscreens which may be formulated into the compositions of the instant invention are those selected from among: aminobenzoic acid, amyldimethyl PABA, cinoxate, diethanolamine p-methoxycinnamate, digalloyl trioleate, dioxybenzone, 2-ethoxyethyl p-methoxycinnamate, ethyl 4-bis(hydroxypropyl)aminobenzoate, 2-ethylhexyl-2-cyano-3,3-diphenylacrylate, ethylhexyl p-methoxycinnamate, 2-ethylhexyl salicylate, glyceryl aminobenzoate, homomenthyl salicylate, homosalate, 3-imidazol-4-ylacrylic acid and ethyl ester, methyl anthranilate, octyldimethyl PABA, 2-phenylbenzimidazole-5-sulfonic acid and salts, red petrolatum, sulisobenzone, titanium dioxide, triethanolamine salicylate, N, N, N-trimethyl-4-(2-oxoborn-3-ylidene methyl)anillinium methyl sulfate, and mixtures thereof.

Similarly preferred sunscreens active in the UV-A and/or UV-B range include:
p-aminobenzoic acid,
oxyethylene (25 mol) p-aminobenzoate,
2-ethylhexyl p-dimethylaminobenzoate,
ethyl N-oxypropylene p-aminobenzoate,
glycerol p-aminobenzoate,
4-isopropylbenzyl salicylate,
2-ethylhexyl 4-methoxycinnamate,
methyl diisopropylcinnamate,
isoamyl 4-methoxycinnamate,
diethanolamine 4-methoxycinnamate,
3-(4'-trimethylammunium)-benzyliden-bornan-2-one methylsulfate,
2-hydroxy-4-methoxybenzophenone,
2-hydroxy-4-methoxybenzophenone-5-sulfonate,
2,4-dihydroxybenzophenone,
2,2',4,4'-tetrahydroxybenzophenone,
2,2'-dihydroxy-4,4'dimethoxybenzophenone,
2-hydroxy-4-n-octoxybenzophenone,
2-hydroxy-4-methoxy-4'-methoxybenzophenone,
a-(2-oxoborn-3-ylidene)-tolyl-4-sulfonic acid and soluble salts thereof,
3-(4'-sulfo)benzyliden-bornan-2-one and soluble salts thereof,
3-(4'methylbenzylidene)-d,1-camphor,
3-benzylidene-d,1-camphor,
benzene 1,4-di(3-methylidene-10-camphosulfonic) acid and salts thereof (the product Mexoryl SX described in U.S. Patent No. 4,585,597 issued to Lange et al. on April 29, 1986),
urocanic acid,
2,4,6-tris[p-(2'-ethylhexyl-1'-oxycarbonyl)-anilino]-1,3,5-triazine,
2-[(p-(tertiobutylamido)anilino]-4,6-bis-[(p-(2'-ethylhexyl-1'-oxycarbonyl)anilino]-1,3,5-triazine,
2,4-bis {[4-(2-ethyl-hexyloxy)]-2-hydroxy]-phenyl}-6-(4-methoxy-phenyl)-1,3,5-triazine ("TINOSORB S" marketed by Ciba),
the polymer of N-(2 et 4)-[(2-oxoborn-3-yliden)methyl]benzyl]-acrylamide,
1,4-bisbenzimidazolyl-phenylen-3,3',5,5'-tetrasulfonic acid and salts thereof,
the benzalmalonate-substituted polyorganosiloxanes,
the benzotriazole-substituted polyorganosiloxanes (Drometrizole Trisiloxane),
dispersed 2,2'-methylene-bis-[6-(2H-benzotriazol-2-yl)-4-(1,1,3,3-tetramethylbutyl)phenol] such as that marketed under the trademark MIXXIM BB/100 by Fairmount Chemical, or micronized in dispersed form thereof such as that marketed under the trademark TINOSORB M by Ciba-Geigy, and
solubilized 2,2'-methylene-bis-[6-(2H-benzotriazol-2-yl)-4-(methyl)phenol] such as that marketed under the trademark MIXXIM BB/200 by Fairmount Chemical.

Typically preferred among the subject sunscreens are one or more of the following: octyl salicylate, octocrylene, and oxybenzone. Combinations of one of more of these sunscreens is similarly preferred.

The dibenzoyl methane derivatives other than avobenzone are also preferred sunscreens according to the present invention. These are described, for example, in FR-2,326,405, FR-2,440,933 and EP-0,114,607, hereby expressly incorporated by reference.

More preferred dibenzoyl methane sunscreens other than avobenzone include (whether singly or in any combination):
2-methyldibenzoylmethane
4-methyldibenzoylmethane
4-isopropyldibenzoylmethane
4-tert.-butyldibenzoylmethane
2,4-dimethyldibenzoylmethane
2,5-dimethyldibenzoylmethane
4,4'-diisopropyldibenzoylmethane
4,4'-dimethoxydibenzoylmethane
2-methyl-5-isopropyl-4'-methoxydibenzoylmethane
2-methyl-5-tert.-butyl-4'-methoxydibenzoylmethane
2,4-dimethyl-4'-methoxydibenzoylmethane
2,6-dimethyl-4-tert.-butyl-4'-methoxydibenzoylmethane

The subject at least one UV-A and/or UV-B sunscreen is advantageously formulated into the compositions of the invention in amounts ranging from about 0.01% to about 10%, and preferably from about 0.1 % to about 6%, by weight thereof. Of course, depending upon the nature of the particular formulation, higher or lower amounts may be suitable.

Concordantly, this invention features the use of glass microspheres as above defined in a topically applicable cosmetic/dermatological sunscreen product for UV-photoprotecting human skin, scalp, lips, mucous membranes and/or hair against the damaging effects of ultraviolet irradiation for improving the aesthetics and SPF values thereof.

Also featured are concordantly UV-photoprotecting and artificial or sunless tanning compositions comprising those constituents (a) and (b) as indicated above, together with an effective amount of at least one artificial/sunless tanning agent, notably dihydroxyacetone or DHA.

To date, a wide variety of artificial tanning agents has been developed. Artificial tanners provide the highly sought-after tanning or darkening response once only available through harmful exposure to ultraviolet radiation. DHA, in particular, has been widely utilized in cosmetics to accomplish artificial tanning of the skin. Proteins of the epidermis have a very high concentration of arginine, lysine, and histidine and the reaction of skin with DHA to produce an artificial tan takes advantage of this fact. The tanning reaction proceeds through combination with free amino groups in skin proteins, and particularly by combination of DHA with the free guanido group in arginine.

Preferred among those artificial tanners which are useful in the compositions of the instant invention are those selected from the group comprising: allose, alpha hydroxy substituted ketones such as dihydroxyacetone, altrose, arabinose, erythrose, fructose, galactose, glucose, glyceraldehyde, indoles, lactose, mannose, reose, ribose, pentose, sucrose, tallose, xylose, and mixtures thereof.

Most preferred among these artificial/sunless tanners which are useful in the compositions of the present inventions is dihydroxyacetone. In this respect, it should be appreciated that DHA is not at all easy to formulate, is particulary sensitive and compositions comprised thereof tend to be quite unstable over time (as DHA tolerates but few raw materials, e.g., carbomers).

The compositions of the present invention can be formulated into a wide variety of product types, including creams, dispersions, emulsions (oil-in-water, water-in-oil, water-in-oil-in-water, and oil-in-water-in-silicone), gels, ointments, lotions, milks, mousses, sprays, tonics, and the like.

The topical cosmetic compositions of the present invention typically comprise a carrier (vehicle or diluent) or mixture of carriers. The carrier should be cosmetically and/or pharmaceutically acceptable, which reflects that the carrier is suitable for topical application onto the skin, has good aesthetic properties, is compatible with any other components, and will not cause any untoward safety or toxicity concerns. The carriers and additional components used to formulate such products vary with the product type and may be routinely chosen by one skilled in the art. The following is a description of some of these carriers and additional components.

The compositions of the present invention can comprise a carrier, or a mixture of carriers, suitable for topical application onto human skin. The carriers typically constitute from about 0.5% to about 99.5% by weight, preferably from about 5.0% to about 99.5% by weight, more preferably from about 10.0% to about 98.0% by weight, of the composition. As used herein, the phrase "suitable for topical application onto human skin" reflects that the carrier does not damage or negatively affect the aesthetics of or cause irritation to human skin.

Carriers suitable for use with the present invention include, for example, those used in the formulation of a wide variety of product types, including creams, dispersions, emulsions, gels, lotions, milks, mousses, sprays, and tonics.

The carriers used herein can include a wide range of components conventionally used in cosmetic/dermatological compositions. The carriers can contain a solvent to dissolve or disperse the polymer. The carriers can also contain a wide variety of additional materials including, but not limited to, esters (such as isopropyl myristate), halogenated hydrocarbons (such as freons), hydrocarbons (such as decene, hexane, and isobutane), linalool, and volatile silicon derivatives (especially siloxanes such as phenyl pentamethyl disiloxane, methoxypropyl heptamethyl cyclotetrasiloxane, chloropropyl pentamethyl disiloxane, hydroxypropyl pentamethyl disiloxane, octamethyl cyclotetrasiloxane, decamethyl cyclopentasiloxane, cyclomethicone, dimethicone), and mixtures thereof.

Mousses and aerosol sprays can also include any of the conventional propellants to deliver the material as a foam, in the case of a mousse, or as a fine, uniform spray, in the case of an aerosol spray. Examples of suitable propellants include materials such as hydrofluorinated compounds, dichlorodifluoromethane, difluoroethane, dimethylether, isobutane, n-butane, propane, or trichlorofluromethane. A tonic or spray product having a low viscosity may also include an emulsifying agent. Examples of suitable emulsifying agents are anionic surfactants, cationic surfactants, nonionic surfactants, and mixtures thereof. Fluorosurfactants are especially preferred, particularly if the product is a preferred spray composition and most especially if it is a spray composition having a relatively low level of volatile organic solvents, such as alcohols, and relatively high levels of water (i.e., in excess of about 10%, by weight, water). If such an emulsifying agent is included, it is preferably present at a level of from about 0.01 % to about 7.5% by weight of the composition. The level of propellant can be adjusted as desired, but is generally from about 3% to about 30% by weight of mousse compositions and from about 15% to about 50% by weight of the aerosol spray compositions.

Suitable spray compositions are well known in the art and include conventional, non-aerosol pump sprays, i.e., "atomizers," aerosol containers or cans having propellant, as described above, and also pump aerosol containers utilizing compressed air as the propellant. Pump aerosol containers are disclosed, for example, in U.S. Patent No. 4,077,441, issued to Olofsson on March 7, 1978, and U.S. Patent No. 4,850,517, issued to Ter Stege on July 25, 1989, both incorporated herein by reference.

A wide variety of additional components can be employed in the topical cosmetic/dermatological compositions herein. The compositions of the present invention can comprise a safe and effective amount of a pharmaceutical additive or adjuvant. The phrase "safe and effective" connotes an amount of an active agent high enough to significantly or positively modify the condition to be treated, but low enough to avoid serious side effects (at a reasonable benefit/risk ratio) within the scope of sound medical judgment. A safe and effective amount of the pharmaceutical active agent will vary with the specific active species, the ability of the composition to penetrate the active species through the skin, the amount of composition to be applied, the particular condition being treated, the age and physical condition of the patient being treated, the severity of the condition, the duration of the treatment, the nature of concurrent therapy, and like factors.

Useful pharmaceutical active agents which may be conjointly administered according to in the present invention include antimicrobial drugs: antibacterials, antifungals, antiprotozoans, and antivirals. Antimicrobial drugs preferred for inclusion in the compositions of the present invention comprise pharmaceutically acceptable salts of β-lactam drugs, amanfadine, amikacin, capreomycin, chlorhexidine, chlortetracycline, ciprofloxacin, clindamycin, doxycycline, erythromycin, ethambutol, gentamicin, kanamycin, lineomycin, methacycline, methenamine, metronidazole, miconazole, minocycline, neomycin, netilmicin, norfloxacin, oxytetracycline, paramomycin, pentamidine, quinolone drugs, streptomycin, tetracycline, tobramycin, and triclosan.

The subject cosmetic/dermatological compositions can contain various emulsifiers when formulated as emulsions. These emulsifiers are useful for emulsifying the various carrier components of the compositions herein. Suitable emulsifiers can include any of a wide variety of nonionic, cationic, anionic, and zwitterionic emulsifiers disclosed in the prior patents and other references. See McCutcheon's, **Detergents and Emulsifiers**, North American Edition (1986), published by Allured Publishing Corporation; U.S. Patent No. 5,011,681, issued to Ciotti et al. on April 30, 1991; U.S. Patent No. 4,421,769, issued to Dixon et al. on December 20, 1983; and U.S. Patent No. 3,755,560, issued to Dickert et al. on August 28, 1973. These four publications are incorporated herein by reference in their entirety.

Suitable emulsifier types include acyl lactylates, alkyl phosphates, carboxylic acid copolymers, esters and ethers of glucose, esters of glycerin, esters of propylene glycol, esters of sorbitan anhydrides, esters of sorbitol, ethoxylated ethers, ethoxylated alcohols, fatty acid amides, fatty acid esters of polyethylene glycol, fatty esters of polypropylene glycol, polyoxyethylene fatty ether phosphates, soaps and mixtures thereof.

Preferred emulsifiers can include, but are not limited to, ceteareth-20, ceteth-10, cetyl phosphate, diethanolamine cetyl phosphate, glyceryl stearate, PEG-100 stearate, polyethylene glycol 20 sorbitan monolaurate, polyethylene glycol 5 soya sterol, polysorbate 60, polysorbate 80, potassium cetyl phosphate, PPG-2 methyl glucose ether distearate, steareth-20, and mixtures thereof.

Typically preferred among these emulsifiers which are useful in the compositions of the present inventions is PPG-2 isoceteth-20 acetate (described in U.S. Patent No. 4,559,226, issued to Fogel et al.).

The subject cosmetic/dermatological compositions can also contain various emollients. Examples of suitable emollients include, but are not limited to, highly branched hydrocarbons, non-polar carboxylic acid and alcohol esters, volatile and non-volatile silicone oils, and mixtures thereof. See, U.S. Patent No. 4,919,934, issued to Deckner et al. on April 24, 1990, which is incorporated by reference in its entirety.

Typically preferred among these emollients which are useful in the compositions of the present inventions are one or more of the following: octyldodecyl neopentanoate and propylene glycol isoceteth-3 acetate.

A variety of additional components can be incorporated into the subject cosmetic/dermatological compositions. Non-limiting examples of these additional components include cationic polymers and thickeners, chelators, gums and thickeners, low pH thickening agents, polymers for enhancing the film-forming properties and substantivity of the composition, sequestrants, humectants, skin penetrating aids, suspending agents, vitamins and derivatives thereof, preservatives and aesthetic components.

Exemplary preservatives, which are conventional in this art and which prevent or retard microbial growth and thus protect cosmetic products from spoilage, are set forth at **CFTA International Cosmetic Ingredient Dictionary and Handbook**, seventh edition, **2**, 1654 (1997).

The cosmetic/dermatological compositions of the present invention are administered in conventional fashion to provide the desired benefit. Such methods of use generally involve topical application of an effective amount of the composition onto the skin, which then is allowed to remain until absorbed into or removed from the skin.

In order to further illustrate the present invention and the advantages thereof, the following specific examples are given, it being understood that same are intended only as illustrative and in nowise limitative.

In said examples to follow, all parts and percentages are given by weight, unless otherwise indicated.

### EXAMPLE 1:

To determine the level of improvement in SPF by the addition of borosilicate glass beads a series of formulations containing only octylmethoxycinnamate ("OMC") and/or titanium dioxide as sunscreen actives, with and without borosilicate beads were evaluated by *in vitro* SPF. *In vitro* SPF testing is a good predictor of *in vivo* efficacy, and has been published in B.L. Diffey & J. Robson, "A new substrate to measure sunscreen protection factors throughout the ultraviolet spectrum," **J. Soc. Cosmet. Chem., 40**, pp. 127-133 (May/June 1989).

The following results were obtained:

| **FORMULATION** | | **SPF VALUE** |
|---|---|---|
| 4% | Borosilicate (no sunscreen) | 2.2 |
| 5% | OMC | 6.6 |
| 5% | OMC + 4% borosilicate | 10.9 |
| 5% | TiO₂ | 6.4 |
| 5% | TiO₂ + 4% borosilicate | 9.2 |
| 2.5% | OMC + 2.5% TiO₂ | 9.8 |
| 2.5% | OMC + 2.5% TiO₂ + 4% borosilicate | 16.3 |

These results are also reported in the accompanying Figure 1 (Fig 1) of Drawing.

### EXAMPLE 2:

The following UV-sunscreen oil-in-water emulsion containing 3% by weight of calcium aluminum borosilicate glass beads (LUXSIL® cosmetic microspheres) was formulated:

| **Phase** | **Ingredient** | **%** |
|---|---|---|
| A | Water | to 100% |
| | Humectant | 6 |
| | Panthenol | 1 |
| | Preservative | 0.6 |
| | Thickener/gum | 0.1 |
| B1 | Avobenzone | 3 |
| | Octocrylene | 10 |
| | Oxybenzone | 6 |
| | Octyl Salicylate | 5 |
| | Preservative | 0.8 |
| | Emulsifier | 2 |
| | Emollient | 10 |
| | Emulsifier/thickener | 0.15 |
| B2 | Calcium Aluminum Borosilicate | 3 |
| C | Silicone Emollient | 4 |
| D | Vitamin E | 0.1 |

### Procedure:

The ingredients of Phase A were introduced into a main tank and heated with homogenization to 80°-85°C. The ingredients of Phase B1 were combined in a side kettle and heated with moderate propeller mixing to 80°C. When melted, Phase B2 (calcium aluminum borosilicate) was slowly added to the side kettle with homogenization to fully disperse the particles in the oil phase. The emulsion was formulated by slowly adding the oil phase to the water phase (side kettle to the main kettle) with increased homogenization. It was mixed for 15 minutes. Cooling was then initiated and homogenization was decreased. When the contents of the main tank reached a temperature of 40°C, Phase C was added to the tank. At 35 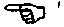 C, Phase D was added to the tank.

### EXAMPLE 3

The procedure of Example 2 is repeated to formulate another oil-in-water emulsion, but in this instance containing 3% by weight of sodium borosilicate glass beads having a mean granulometry of 20 µm (manufactured by PQ Corporation).

### EXAMPLE 4:

*In vitro* SPF testing of a sunscreen lotion containing 3% calcium aluminum borosilicate LUXSIL® glass microbeads provided an average SPF value of 119.
A control lotion was compared that was identical to the test formulation except that it did not contain the borosilicate beads. Its average SPF was 47.

Each patent, patent application, publication and literature article/report cited or indicated herein is hereby expressly incorporated by reference.

While the invention has been described in terms of various specific and preferred embodiments, the skilled artisan will appreciate that various modifications, substitutions, omissions, and changes may be made without departing from the spirit thereof. Accordingly, it is intended that the scope of the present invention be limited solely by the scope of the following claims, including equivalents thereof.

## Claims

1. A topically applicable cosmetic/dermatological sunscreen composition suited for UV-photoprotecting the skin, scalp, lips, mucous membranes and/or hair against the damaging effects of ultraviolet irradiation, **characterized in that** it comprises (a) at least one UV-A sunscreen and/or at least one UV-B sunscreen and (b) at least glass microspheres, formulated into (c) a topically applicable, cosmetically/dermatologically acceptable vehicle, diluent or carrier therefor.

2. The cosmetic/dermatological sunscreen composition as defined by Claim 1, wherein said glass microspheres comprising a high slip, non-absorbent, non-porous white powder, essentially uniformly spheroidal.

3. The cosmetic/dermatological sunscreen composition as defined by Claim 1, wherein said glass microspheres having a mean particle size of up to 70 µm.

4. The cosmetic/dermatological sunscreen composition as defined by Claim 3, wherein said glass microspheres having a mean particle size of about 20 µm or less.

5. The cosmetic/dermatological sunscreen composition as defined by Claim 4, wherein said glass microspheres having a mean particle size on the order of 9-13 µm.

6. The cosmetic/dermatological sunscreen composition as defined by Claim 1, wherein said glass microspheres comprising calcium aluminum borosilicate.

7. The cosmetic/dermatological sunscreen composition as defined by Claim 1, wherein glass microspheres comprising sodium borosilicate.

8. The cosmetic/dermatological sunscreen composition as defined by Claim 1, wherein said sunscreen (a) and said glass microspheres (b) being essentially homogeneously and uniformly distributed throughout said topically applicable, cosmetically/dermatologically acceptable vehicle, carrier or diluent (c).

9. The cosmetic/dermatological sunscreen composition as defined by Claim 1, wherein said glass microspheres comprising from about 0.1% to 10% by weight thereof.

10. The cosmetic/dermatological sunscreen composition as defined by Claim 1, wherein said glass microspheres comprising from about 1% to 5% by weight thereof.

11. The cosmetic/dermatological sunscreen composition as defined by Claim 1, comprising at least one hydrophilic organic UV-sunscreen and/or at least one lipophilic organic UV-sunscreen.

12. The cosmetic/dermatological sunscreen composition as defined by Claim 11, comprising at least one dibenzoylmethane, anthranilate, benzophenone, p-aminobenzoic acid, camphor, cinnamate, salicylate, β,β-diphenylacrylate, triazine, benzimidazole, bis-benzoazolyl, methylene bis-(hydroxyphenylbenzotriazole), polymer or silicone sunscreen, or mixture thereof.

13. The cosmetic/dermatological sunscreen composition as defined by Claim 12, comprising at least one avobenzone, octocrylene, oxybenzone, and/or octyl salicylate sunscreen.

14. The cosmetic/dermatological sunscreen composition as defined by Claim 1, comprising at least one metal oxide sunscreen.

15. The cosmetic/dermatological sunscreen composition as defined by Claim 1, further comprising an effective amount of at least one artificial/sunless tanning agent.

16. The cosmetic/dermatological sunscreen composition as defined by Claim 15, said at least one artificial/sunless tanning agent comprising dihydroxyacetone.

17. The cosmetic/dermatological sunscreen composition as defined by Claim 1, formulated as a cream, dispersion, emulsion, gel, ointment, lotion, milk, mousse, spray, or tonic.

18. A topically applicable cosmetic/dermatological sunscreen oil-in-water emulsion suited for UV-photoprotecting the skin, scalp, lips, mucous membranes and/or hair against the damaging effects of ultraviolet irradiation, comprising (a) at least one UV-A sunscreen and/or at least one UV-B sunscreen and (b) at least glass microspheres, formulated into (c) a topically applicable, cosmetically/dermatologically acceptable vehicle, diluent or carrier therefor.

19. The topically applicable cosmetic/dermatological sunscreen oil-in-water emulsion as defined by Claim 18, said glass microspheres comprising a high slip, non-absorbent, non-porous white powder, essentially uniformly spheroidal.

20. The topically applicable cosmetic/dermatological sunscreen oil-in-water emulsion as defined by Claim 19, comprising hollow glass microspheres.

21. Use of glass microspheres as defined in any claims 1 to 8 in a topically applicable cosmetic/dermatological sunscreen product for UV-photoprotecting human skin, scalp, lips, mucous membranes and/or hair against the damaging effects of ultraviolet irradiation for improving the aesthetics and SPF values thereof.

22. Use as defined by Claim 20, wherein said glass microspheres comprise a high slip, non-absorbent, non-porous white powder, essentially uniformly spheroidal.
